Europäisches Patentamt

European Patent Office

Office européen des brevets

(11) Publication number: **0 124 365**
**B1**

# EUROPEAN PATENT SPECIFICATION

(45) Date of publication of patent specification: **15.10.86**

(21) Application number: **84302851.5**

(22) Date of filing: **27.04.84**

(51) Int. Cl.⁴: **A 61 F 13/00,** A 61 F 5/455,
A 41 B 13/02, A 61 F 13/16

(54) Absorbent structure with reservoirs and a channel.

(30) Priority: **29.04.83 US 489980**

(43) Date of publication of application:
**07.11.84 Bulletin 84/45**

(45) Publication of the grant of the patent:
**15.10.86 Bulletin 86/42**

(84) Designated Contracting States:
**AT BE CH DE FR IT LI LU NL SE**

(56) References cited:
**GB-A-1 133 104**
**GB-A-2 087 730**
**US-A-3 512 185**
**US-A-3 736 931**
**US-A-3 888 257**
**US-A-4 184 498**

(73) Proprietor: **PERSONAL PRODUCTS COMPANY**
**Van Liew Avenue**
**Milltown New Jersey 08850 (US)**

(72) Inventor: **Holtman, Dennis C.**
**Box 125 RD No. 5 Old Clinton Road**
**Flemington, NJ 08822 (US)**

(74) Representative: **Jones, Alan John et al**
**CARPMAELS & RANSFORD**
**43 Bloomsbury Square**
**London, WC1A 2RA (GB)**

Courier Press, Leamington Spa, England.

## Description

### Background of the Invention

Disposable absorbent structures such as disposable diapers, sanitary napkins and incontinence pads have met with increased commercial acceptance in recent years primarily because of their convenience. Many different constructions have been proposed and used and some have met with widespread commercial success in spite of certain inadequacies in functional properties.

One of the most serious prior art problems has been the inability to provide a suitable construction that can accept a large void of body fluids. This is particularly true of the adult incontinent whether the incontinent be active and working or ill and bedridden. Various attempts have been made to provide special structures to absorb a large body fluid void. These include U.S. Patent 3,441,024 ot H. J. Ralph, U.S. Patent 3,747,602 to H. J. Ralph, U.S. Patent 3,968,798 to K. C. Hokanson, and U.S. Patent 4,067,366 to R. L. Johnson. While these various constructions were designed to assist the adult incontinent, the problem of providing an overall disposable absorbent structure which will handle a full volume discharge of urine without leakage still remains.

Particularly when the incontinent is an adult, disposable strutures generally have not accepted and held a full volume discharge of urine without leakage onto clothing.

Published British patent application No. 2087730 to D. Holtman discloses an absorbent structure for receiving and containing body fluids, comprising a generally rectangular, loosley compacted, cellulosic fibrous batt having a moisture impermeable backing sheet on one side and a fabric cover which covers at least the side of the absorbend batt opposite the backing sheet, the batt containing on one of its surfaces a reservoir having a capacity of at least 10 cc, which is substantially centered on the longitudinal axis of the batt, and which is formed by compression of the cellulosic fibers.

Although advances have been made in the sanitary napkin art to handle a full discharge of menstrual fluid (see U.S. Patent 4,184,498 to P. Franco), disposable structures which will handle a complete urine void by an adult are absent. The present invention provides an absorbent structure which accepts and holds a complete urine void by an adult and which may be used as an infant diaper, an adult incontinence device, a sanitary napkin, an incontinence pad, or the like.

The present invention provides an absorbent structure of the type disclosed in GB—A—2 087 730, which is characterised in that:
the batt contains on said one of its surfaces at least a second reservoir having a capacity of at least 10 cc; the reservoirs are spaced apart and connected by a connecting channel; the channel depth is at least half the depth of the reservoirs; the reservoirs and the length of the connecting channel are substantially centred on the longitudinal axis of the batt; and the reservoirs and connecting channel are formed by compression of the cellulosic fibres.

Preferably; the length of the batt is at least 1.5 times its width; and the width of the batt does not exceed 12.7 cm (5 inches).

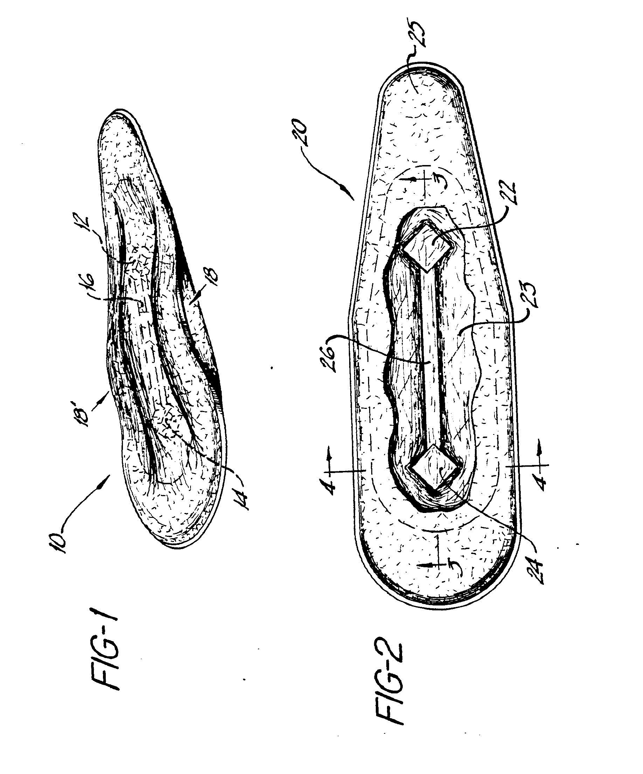

The two reservoirs and the channel act in concert to provide a three-dimensional product unlike the two dimensional products of the prior art. When the structure of the present invention is worn by the user the legs of the user apply pressure to the structure against each side, the structure selectively collapses at the channel more so than at the points of the reservoirs. This provides a boat shape by the back and front coming up (see Figure 1) and the reservoirs staying open and available. The selective collapsing of the channel keeps the strike zone on the longitudinal centreline. By keeping the reservoirs on the longitudinal centerline, maximum use is made of this reservoir's capacity.

The absorbent structure may be incorporated in a disposable diaper such as for an infant or in an adult diaper with a larger liquid holding capacity. Furthermore, the absorbent structure may be incorporated in a sanitary napkin as well as in an incontinence pad. In each instance the opening of one reservoir or the channel is close enough to the void zone to allow immediate acceptance by the structure of the body fluid discharge. Generally, each reservoir will hold at least 75 percent of the body fluid discharged in one second.

The reservoirs and channel are created by compression of the loosely-compacted, cellulosic fibers. The compaction may be carried out by application of pressure in the shape desired for the reservoirs and channel. This may be done by a stamping-type operation or by an embossing roll.

### Brief Description of the Drawings

Fig. 1 is a perspective view of one embodiment of the present invention;

Fig. 2 is a top view of the embodiment of Figure 1 with a portion broken away for clarity;

Fig. 3 is a cross-sectional view taken along line 3—3 of Figure 2;

Fig. 4 is a cross-secitonal view taken along line 4—4 of Figure 2;

Figs, 5, 6, 7 and 8 are top views of different embodiments of the present invention.

Fig. 9 is a perspective view of a diaper incorporating the absorbent structure of the present invention; and

Fig. 10 is a perspective view of the diaper of Figure 9 as it appears when worn.

### Detailed Description of the Invention

While this invention is susceptible of embodiment in many different forms, there is shown in the drawings, and herein will be described in detail, preferred embodiments of the invention and modifications thereof. It is understood that the present disclosure is to be considered as an exemplification of the invention and is not intended to limit the invention to the embodiments illustrated. The scope of the invention will be pointed out in the appended claims.

Referring to the drawings and particularly to Figure 1, an absorbent structure 10 is provided with reservoirs 12 and 14 connected by a channel 16. The view in Figure 1 is a perspective view of the absorbent structure as it would appear while being worn. Preferably, the underside of the structure has at least one adhesive strip whereby the structure can be fastended to the underwear of the wearer. The narrow portion of the pad is the back portion whereas the wider portion of the pad would be worn toward the front. The pad is satisfactory for use as a urinary pad or as a menstrual fluid pad. When the absorbent structure is being worn the thighs of the wearer would press at points 18 and 18' to cause the pad to form a boat-like shape where the sides of the pad are higher than the central portion. This assists in preventing leakage by any runover of fluid over the sides.

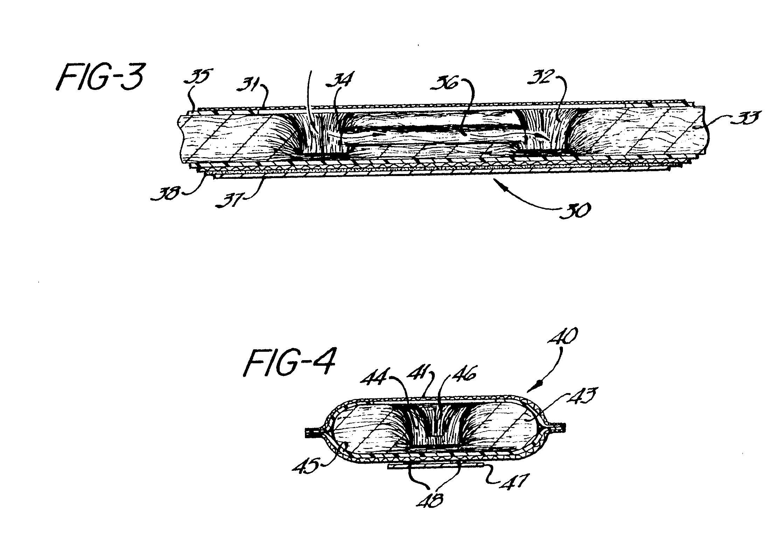

Figure 2 is a top view of the pad of Figure 1 showing an absorbent structure 20 having a back reservoir 22 and a front reservoir 24 connected by a channel 26. A portion of the opening is broken away to show the absorbent batt 23. A permeable facing 25 covers the upper surface. The sides and lower surface are protected by a moisture barrier such as a polyethylene backing.

Figure 3 provides a cross-sectional view 30 of the absorbent structure of Figure 2 along lines 3—3. The absorbent structure has reservoirs 32 and 34 connected by channel 36. If the pad were to be used as a urinary pad, it could be expected that the flow of urine into the pad would follow the direction of the arrows. The absorbent structure has an absorbent batt of loosley-compacted cellulosic fibers 33 and is provided with a moisture barrier 35 which comes up around the sides and covers a portion of the top over which is placed a liquid permeable facing 31 which may be in the form of a nonwoven such as polyester or other suitable fabric. The under surface is provided with an adhesive strip 38 to which is attached a release liner 37. Upon removal of the liner the adhesive is available for fastening to the clothing of the wearer.

Figure 4 is a cross-sectional view 40 of the absorbent strucutre of Figure 2 along lines 4—4. This figure particularly illustrates the rlelationship of the depth of the channel in relation to the reservoirs. As can be seen, the reservoir 44 is deeper than the channel 46. This is a preferred embodiment, although the channel can be as deep as the reservoirs. The cellulosic batt 43 is surrounded most of the way except for an opening on top by a moisture barrier 45. The facing 41 is used as an overwrap to wrap substantially all of the product. Release liner 47 is removed to provide the adhesive 48 for fastening to the clothing.

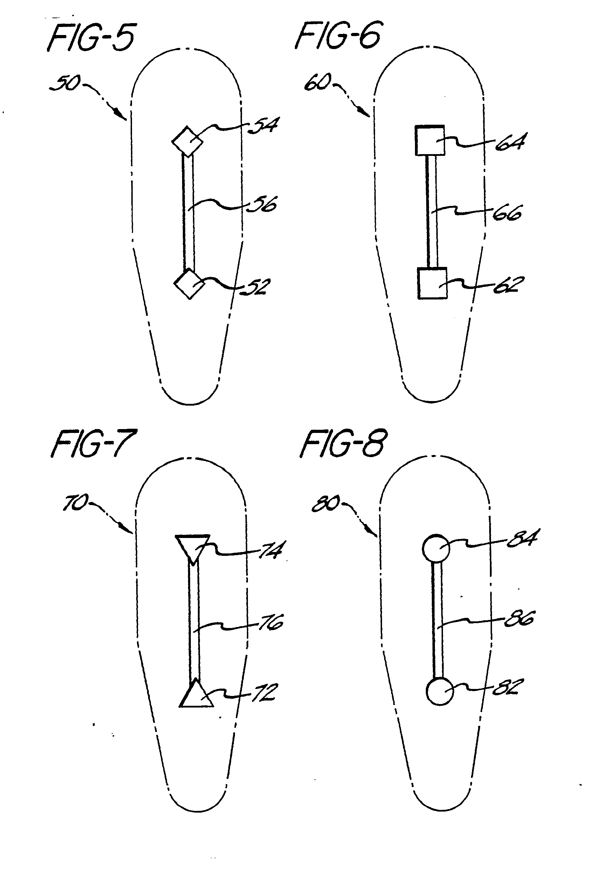

Figures 5, 6, 7 and 8 illustrate absorbent structures 50, 60, 70 and 80 respectively having different shapes for the reservoirs. These are merely illustrative and other shapes and size relationships may be used satisfactorily. In each case, the numeral ending in 2 and 4 denotes the reservoirs whereas the numeral ending in 6 depicts the channel.

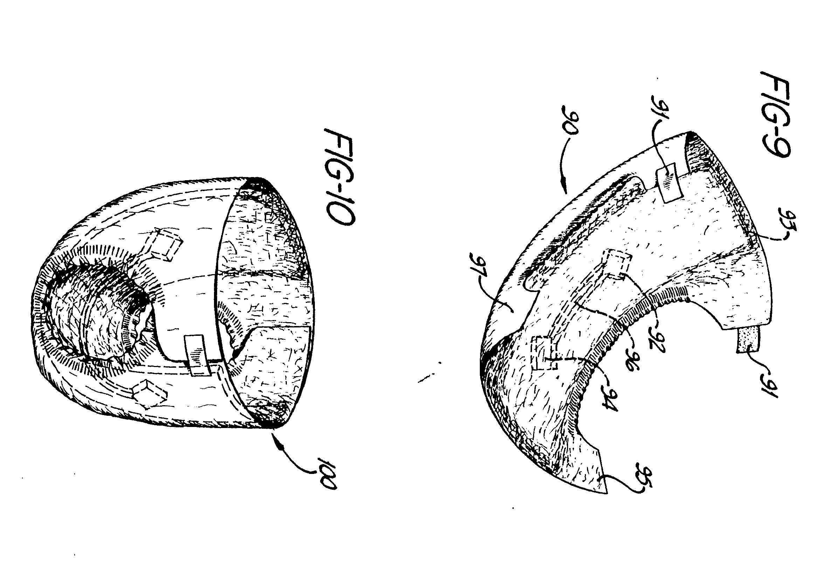

The absorbent structure of the present invention may also be used in the form of a diaper. Such a diaper 90 is illustrated in Figure 9. The cellulosic batt 93 is provided with reservoirs 92 and 94 with the connecting channel 96. Surrounding the cellulosic batt is a moisture-impermeable backing 97 and a moisture-permeable facing 95. The facing and backing are laminated in the margins and elastic is placed in the diaper for suitable fit. When the diaper is worn, tape tabs 91 secure the diaper about the wearer. In Figure 10, the diaper 100 is illustrated as it would appear on a wearer.

The loosely-compacted cellulosic fibrous batt used in the absorbent structure of the present invention is formed from wood pulp fibers, rayon fibers, cotton linters or mixtures thereof. The batt is primarily held together by interfiber bonds requiring no added adhesive. In some instances the batt may contain synthetic fusible fibers such as polyethylene, polypropylene. The batt is a bulk density coherent web of loosely-compacted cellulosic fibers, preferably cumminuted wood pulp fibers in the form of so-called "fluff". When the reservoirs and channels are created in the batt, a compressing or compaction process is used. The compression should be sufficient to create reservoirs of sufficient depth to hold at least 10 cc. The base of each of the reservoirs is a densified fibrous region wherein the fibers are compacted. The densified region quickly absorbs the body fluid and commences distribution of the fluid to other parts of the cellulosic batt particularly along the connecting channels. The channel is at least half the depth of the reservoirs. Although the base of the channel may not have as high a density as the base of a reservoir, there is at least some densifying of the fibers at the base of the channel. This densifying assists in transporting the liquid from one reservoir to another and to other parts of the cellulosic fibrous batt. The location and spacing of the reservoirs may be customized to optimize conditions for particular use. In the instance of urinary pads, it is desirable for the reservoirs to be placed at least 5.08 cm (two inches) apart but preferably not more than about 15.24 cm (six inches) apart. It has been found that, even in use for a menstrual pad where the reservoirs may be relatively close together, at least 1.27 cm (one-half inch) between the reservoirs should be provided for the channel and preferably at least about 2.54 cm (an inch) is suitable for the length of the channel. In all instances, the reservoirs and the channel should lie substantially on the longitudinal axis of the absorbent structure.

Although the absorbent structure is substantially rectangular, it can assume other shapes such as those illustrated in the drawings. One primary benefit afforded by the combination of the substantially rectangular shape of the reservoirs and the connecting channel is that when the structure is worn, the thighs, whether they be those of an infant or an adult, place pressure on the sides and the sides come up in the central portion to provide a boat-like configuration. Such a configuration is highly desirable to prevent leakage of body fluids at the side edges.

The absorbent structure is provided with a moisture barrier backing on the side away from the one receiving fluids. This barrier may come up around the sides and cover most of the top surface. However, it is necessary that both of the reservoirs and the channels be exposed to the surface for reception of fluid. The top surface or the entire surface may be overwrapped in a fluid permeable material such as a nonwoven fabric. For example, the fabric may be polyester, polyethylene, polypropylene, nylon, rayon. Preferably, the fabric used for the cover is a lightweight fabric in the range of 10.2 to 169.5 g/m² (0.3 to 5 ounces per square yard) and with a density less than 0.2 grams per cc. The most suitable fabrics have unusually high elongation loft softness and drape characteristics. Though the cover is moisture-permeable, it is preferably of the type which after permeation of the moisture prevents strike back of the body fluid when the absorbent structure is approaching saturation.

The body of the cellulosic fibrous batt is substantially more wettable than the cover and tends to draw liquid away from the facing layer. Thus the void occurs and the cover is permeated and the liquid deposited in at least one reservoir. The individual fibers of the batt are extremely wettable and thus draw the liquid into the batt and along the channel to the other reservoir. The base of the reservoir is a densified fibrous region wherein the capillary radius is quite small. Thus, the capillary pressure is increased and the liquid is rapidly distributed to portions of the cellulosic batt. Furthermore, the base of the channel is of a higher density than the immediately surrounding areas except for the reservoir. Thus, this densified fibrous region at the base of the channel provides sufficient capillary pressure to transport the liquid to the other reservoir whereupon the highly densified base of the other reservoir further absorbs the liquid and distributes it. These densified fibrous regions at the base of the reservoirs and the channel provide the maximum capillary pressure because it combines the very low contact angle of the fibers of the batt with the high density of the densified fibers.

The individual fibers of the batt are extremely wettable, generally having liquid-fiber contact angles below about 30° in the optimum embodiment. The wickability or preferential absorptivity of the body of the batt for water is limited, however, by its low density which results in large effective capillary radius for the capillaries between adjacent fibers.

The pressure causing a liquid to enter a cylindrical capillary is expressed by the equation

$$P = \frac{2\,\gamma\,\cos\Theta}{r}$$

wherein

P is the capillary pressure,

γ is the surface tension in the liquid,

Θ is the liquid-fiber contact angle, and

r is the capillary radius.

With a given liquid, the pressure (capillary force) increases with the cosine of the liquid-fiber contact angle (reaching a maximum where the angle is zero) and increases with narrower capillary radii so that narrower capillaires will draw liquid from wider ones.

The relative wickability between the cover layer and the body of the fibrous batt is affected by both the relative densities of the layers and the realtive wettability of the individual fibers in each layer. The individual fibers of the batt have substantially smaller liquid-fiber contact angles than those of the cover layer overcoming the density difference and providing a substantial overall increase in capillary pressure to absorb liquid into the body of the batt.

A densified fiber layer such as that at the base of the reservoirs and the channel provide the maximum capillary pressure because it combines the very low contact angle of the fibers of the batt with the high density (small capillary radius) of the densified fibers.

Thus, when urine is voided into an area in the cover layer, it partially wets a small portion of the cover layer and is passed through the cover layer, almost instantaneously, to the reservoir. It then comes into contact with the body of the batt surrounding the reservoir and the channel and is preferentially absorbed into the body of the batt because of the enhanced wettability of the reservoir and channel and particularly the base of the reservoir and channel. The same is true when the body fluid is a menstrual fluid or other liquid exudate.

In addition, it may be desirable to provide a paper-like densified layer on the side of the batt opposite the reservoir. The paper-like densified layer is formed by a slight moistening of the surface of the batt followed by light compaction using the application of pressure. U.S. Patent No. 3,017, 304 to Burgeni provides such a densified layer in a loosely-compacted cellulosic fibrous batt.

This paper-like densified layer promotes wicking of liquid to all portions of the structure.

It will be understood by those skilled in the art that variations and modifications of the specific embodiments described above may be employed without departing from the scope of the invention as defined in the appended claims.

## Claims

1. An absorbent structure (30) for receiving and containing body fluids, comprising a generally rectangular, loosely compacted, cellulosic fibrous batt (33) having a moisture impermeable backing sheet (35) on one side and a fabric cover (31) which covers at least the side of the absorbent batt (33) opposite the backing sheet (35), the batt (33) containing on one of its surfaces a reservoir (32) having a capacity of at least 10 cc, which is substantially centred on the longitudinal axis of the batt (33), and which is formed by compression of the cellulosic fibers, the structure being characterised in that: the batt (33) contains on said one of its surfaces at least a second reservoir (34) having a capacity of at least 10 cc; the reservoirs (32, 34) are spaced apart and connected by a connecting channel (36); the channel (36) depth is at least half the depth of the reservoirs (32, 34); the reservoirs (32, 34) and the length of the connecting channel (36) are substantially centred on the longitudinal axis of the batt (33); and the reservoirs (32, 34) and connecting channel (36) are formed by compression of the cellulosic fibres.

2. The structure of claim 1 wherein the base of the reservoirs (32, 34) is a densified region of the fibres of the fibrous batt (33).

3. The structure of claim 1 or claim 2, wherein each reservoir (32, 34) is located between the center and one transverse end of the absorbent structure (30).

4. The absorbent structure of any one of claims 1 to 3 wherein the capacity of the reservoirs (32, 34) is sufficient to hold at least 75 percent of the body fluid discharged in one second.

5. The absorbent structure of any one of claims 1 to 4, wherein the surface of the absorbent batt (33) opposite the reservoirs (32, 34) is provided with a paper-like densified skin.

6. The absorbent structure of any one of claims 1 to 5, which is provided with an adhesive strip (38) for securement of the structure when worn.

7. The absorbent structure of any one of claims 1 to 6, wherein the length of the batt (33) is at least 1.5 times its width and the width of the batt (33) does not exceed 12.7 cm (5 inches).

8. The absorbent structure of any one of claims 1 to 7 in the form of a sanitary napkin, a disposable diaper or an incontinence pad.

### Patentansprüche

1. Eine absorbierende Struktur (30) für die Aufnahme und Retention von Körperflüssigkeiten, umfassend eine im allgemeinen rechteckige, locker verdichtete, cellulosehältige faserige Füllung (33) mit einer feuchtigkleitsundurchlässigen Unterlage (35) auf einer Seite und einer Abdecklage (31) aus Textilmaterial, welche wenigstens jene Seite der absorbierenden Füllung (33) bedeckt, welche auf der entgegengesetzten Seite der Füllung (33) wie die Unterlage (35) liegt, wobei die Füllung (33) auf einer inhrer Oberflächen ein Reservoir (32) mit einem Fassungsvermögen von wenigstens 10 cm³ enthält, welches Reservoir im wesentlichen auf der Längsachse der Füllung (33) zentriert ist, und welches Reservoir durch Kompression der Cellulosefasern geformt wird, welche Struktur dadurch gekennzeichnet ist, daß: die Füllung (33) auf der genannten einen inhrer Oberflächen wenigstens ein zweites Reservoir (34) mit einem Fassungsvermögen von wenigstens 10 cm³ enthält; daß die Reservoirs (32, 34) im Abstand voneinander angerordnet sind und miteinander durch einen Verbindungskanal (3) verbuden sind; daß die Tiefe des Kanals (36) wenigstens die Hälfte der Tiefe der Reservoirs (32, 34) ausmacht; daß die Reservoirs (32, 34) un die Längserstreckung des Verbindungskanals (36) im wesentlichen auf der Längsachse der Füllung (33) zentriert sind; und daß die Reservoirs (32, 34) und der Verbindungskanal (36) durch Kompression der Cellulosefasern geformt sind.

2. Die Struktur nach Anspruch 1, worin die Basis der Reservoirs (32, 34) ein verdichteter Bereich der Fasern der faserigen Füllung (33) ist.

3. Die Struktur nach Anspruch 1 oder Anspruch 2, worin jedes Reservoir (32, 34) zwischen dem Mittelpunkt und einem Querende der absorbierenden Struktur (30) angeordnet ist.

4. Die absorbierende Struktur nach einem der Ansprüch 1 bis 3, worin das Fassungsvermögen der Reservoirs (32, 34) ausreicht, um wenigstens 75% der in einer Sekunde agegebenen Körperflüssigkeit aufzunehmen.

5. Die absorbierende Struktur nach einem der Ansprüch 1 bis 4, worin die Oberfläche de absorbierenden Füllung (33), welche den Reservoirs (32, 34) zugekehrt ist, mit einer papierartigen, verdichteten Haut ausgestattet ist.

6. Die absorbierende Struktur nach einem der Ansprüch 1 bis 5, welche mit einem Klebestreifen (38) zum Befestigen der Struktur beim Tragen ausgestattet ist.

7. Die absorbierende Struktur nach einem der Ansprüch 1 bis 6, worin die Länge der Füllung (33) wenigstens das 1,5-fache ihrer Breite ausmacht und worin die Breite der Füllung (33) 12,7 cm (5 Inch) nich überschreitet.

8. Die absorbierende Struktur nach einem der Ansprüch 1 bis 7, in der Form einer hygienischen Binde, einer Wegwerfwindel oder einer Inkontinenzeinlage.

### Revendications

1. Structure absorbante (30) destinée à recevoir et à contenir des fluides corporels, comprenant un matelas fibreux cellulosique (33) faiblement tassé, de forme générale rectangulaire, portant une feuille de doublage imperméable à l'humidité (35) sur une face et un recouvrement d'étoffe (31) quit couvre au moins la face du matelas absorbant (33) que est à l'opposé de la feuille de doublage (35), le matelas (33) contenant, sur une première de ses surfaces, un réservoir (32) ayant une capacité d'au moins 10 cm³ qui est sensiblement centré sur l'axe longitudinal du matelas (33) et qui est formé par compression des fibres cellulosiques, la structure étant caractérisée en ce que: le matelas (33) porte, sur ladite première de ses surfaces, au moins un deuxième réservoir (34) ayant une capacité d'au moins 10 cm³; les réservoirs (32, 34) sont espacés et reliés par un canal de liaison (36); la profondeur du canal (36) est au moins égale à la moitié de la profondeur des réservoirs (32, 34); les réservoirs (32, 34) et la longueur du canal de liaison (36) sont sensiblement centrés sur l'axe longitudinal du matelas (33); et les réservoirs (32, 34) et le canal de liaison (36) sont formés par compression des fibres cellulosiques.

2. Structure selon la revendication 1, dans laquelle le fond des réservoirs (32, 34) est une région densifiée de fibres du matelas fibreux (33).

3. Structure selon la revendication 1 ou la revendication 2, dans laquelle chaque réservoir (32, 34) est situé entre le centre et une extrémité transversale de la structure absorbante (30).

4. Structure absorbante selon une quelconque des revendications 1 à 3, dans laquelle la capacité des réservoirs (32, 34) est suffisante pour contenir au moins 75% du fluide corporel émis en une second.

5. Structure absorbante selon une quelconque des revendications 1 à 4 dans laquelle la surface du matelas absorbant (33) qui est à l'opposé de réservoirs (32, 34) est munie d'une peau densifiée analogue à un papier.

6. Structure absorbante selon une quelconque des revendications 1 à 5, qui est munie d'une bande adhésive (38) servant à fixer la structure lorsqu'elle est posée.

7. Structure absorbante selon une quelconque des revendications 1 à 6, dans laquelle la longueur du matelas (33) est d'au moins 1,5 fois sa largeur et la largeur du matelas (3) n'excède pas 12,7 cm (7 pouces).

8. Structure absorbante selon l'une quelconque des revendications 1 à 7, présentant la forme d'une serviette hygiènique, d'une couche jetable ou une garniture d'incontinence.

*FIG-1*

*FIG-2*

0 124 365

FIG-3

FIG-4

FIG-5

50

54

56

52

FIG-6

60

64

66

62

FIG-7

70

74

76

72

FIG-8

80

84

86

82

*FIG-9*

*FIG-10*